# EUROPEAN PATENT SPECIFICATION

(11) **EP 2 108 395 B1**
(45) Date of publication and mention of the grant of the patent: **17.07.2013**
(21) Application number: 09008924.4
(22) Date of filing: 22.11.2006
(51) Int. Cl.: A61M 15/00

(54) **Aerosol dispensing apparatus**
Vorrichtung zur Abgabe von Aerosol
Appareil de distribution d'aérosol

(30) Priority: 22.11.2005 GB 0523716
(43) Date of publication of application: 14.10.2009
(62) Divisional of application: 08003115.6
(73) Proprietor: Consort Medical PLC, Breakspear Way Hemel Hempstead Hertfordshire HP2 4TZ (GB)
(72) Inventor: Wright, Andrew, Norfolk PE31 7EH (GB); Warby, Richard, Wisbeth, Norfolk PE14 8AF (GB); Allsop, Paul, Norfolk PE30 3XD (GB); Southby, William, Norfolk PE30 3LY (GB); Swain, Martin, Norfolk PE30 5AP (GB); Ingram, Simon, Northampton NN2 6BS (GB); Rockell, Nicholas, Norfolk PE37 7TN (GB)
(74) Representative: Thomson, Neil David

(56) References cited:
- WO-A-2005/044354
- US-A1- 2003 209 239
- US-B1- 6 189 739

## Description

The present invention relates to an aerosol dispensing apparatus, and in particular to such an apparatus designed to dispense metered doses of a product on actuation of a pressurised or pressurisable container.

Aerosol dispensing apparatus for use in dispensing metered doses of product are well known. It has been recognised that there is a need to provide accurate information to a user of a dose-dispensing delivery apparatus concerning the number of doses delivered from, or remaining in, the dispensing apparatus. Without such information, there is a danger that a user will forget how many doses have been delivered and hence take a greater or fewer number of doses than is required. There is also a danger that a user may be unaware that the dispensing apparatus is empty or close to empty. This is especially dangerous where the dispensing apparatus is for use in delivering medicinal compounds for the treatment of chronic or acute symptoms, for example, as in the case of a pressurised metered dose inhaler used for treating asthmatic reactions. A number of devices have been proposed to count the number of doses delivered or remaining in a delivery apparatus. WO95/08484 teaches a dose counting device for use with an aerosol medication dispenser. The device works by translating a non-rotative force on an outer cover into a rotation of an indicator wheel by use of a set of pawls engaged with a set of teeth. The pawls depress and thereby extend circumferentially when the applied force forces them to effect a rotation of the teeth. If the pawls are too stiff relative to the internal spring bias then the medication dispenser may dispense a dose before the pawls flex sufficiently to rotate the indicator wheel; a dose would be delivered without the counter registering it. Alternatively, if the pawls are too flexible relative to the internal spring bias then the pawls may flex sufficiently to rotate the indicator wheel before the medication dispenser has dispensed a dose; a dose would be registered by the counter but not actually delivered.

Further, it is often the tolerance stack-up in the valve of the container and the dose counter that causes the apparatus under or over count.

WO 93/24167 discloses aerosol dispensing apparatus comprising a housing in which a container can be located, an outlet leading from housing, a receiving block having an inlet for receiving an outlet member of the container and a passage through which contents of the container may pass on their way to the outlet when the container is actuated, the housing containing a carriage in which the container is firmly gripped, the carriage being able to slide reciprocally within the housing as the container moves between rest and actuated positions, the carriage comprising a projection, the housing being formed with an upper part which houses at least the container in use and a lower part comprising at least the outlet, the upper and lower parts being conjoined so that the lower part may rotate relative to the upper part.

WO2005/0044354 describes a fluid dispensing device comprising a pre-load means to prevent actuation of a compression pump before a pre-determined force is applied to an actuating lever.

The present invention provides aerosol dispensing apparatus as set out in the appended claims.

The aerosol dispensing apparatus may be a pharmaceutical dispensing device, such as, for example, a pulmonary, nasal, or sub-lingual delivery device. A preferred use of the dispensing apparatus is as a pharmaceutical metered dose aerosol inhaler device. The term pharmaceutical, as used herein, is intended to encompass any pharmaceutical, compound, composition, medicament, agent or product which can be delivered or administered to a human being or animal, for example pharmaceuticals, drugs, biological and medicinal products. Examples include antiallergics, analgesics, bronchodilators, antihistamines, therapeutic proteins and peptides, antitussives, anginal preparations, antibiotics, antiinflammatory preparation, hormones, or sulfonamides, such as, for example, a vasoconstrictive amine, an enzyme, an alkaloid, or a steroid, including combinations of two or more thereof. In particular, examples include isoproterenol [alpha-(isopropylaminomethyl) protocatechuyl alcohol], phenylephrine, phenylpropanolamine, glucagon, adrenochrome, trypsin, epinephrine, ephedrine, narcotine, codeine, atropine, heparin, morphine, dihydromorphinone, ergotamine, scopolamine, methapyrilene, cyanocobalamin, terbutaline, rimiterol, salbutamol, flunisolide, colchicine, pirbuterol, beclomethasone, orciprerlaline, fentanyl, and diamorphine, streptomycin, penicillin, procaine penicillin, tetracycline, chlorotetracycline and hydroxytetracycline, adrenocorticotropic hormone and adrenocortical hormones, such as cortisone, hydrocortisone, hydrocortisone acetate and prednisolone, insulin, cromolyn sodium, and mometasone, including combinations of two or more thereof.

The pharmaceutical may be used as either the free base or as one or more salts conventional in the art, such as, for example, acetate, benzenesulphonate, benzoate, bircarbonate, bitartrate, bromide, calcium edetate, camsylate, carbonate, chloride, citrate, dihydrochloride, edetate, edisylate, estolate, esylate, fumarate, fluceptate, gluconate, glutamate, glycollylarsanilate, hexylresorcinate, hydrobromide, hydrochloride, hydroxynaphthoate, iodide, isethionate, lactate, lactobionate, malate, maleate, mandelate, mesylate, methylbromide, methylnitrate, methylsulphate, mucate, napsylate, nitrate, pamoate, (embonate), pantothenate, phosphate, diphosphate, polygalacturonate, salicylate, stearate, subacetate, succinate, sulphate, tannate, tartrate, and triethiodide, including combinations of two or more thereof. Cationic salts may also be used, for example the alkali metals, e.g. Na and K, and ammonium salts and salts of amines known in the art to be pharmaceutically acceptable, for example glycine, ethylene diamine, choline, diethanolamine, triethanolamine, octadecylamine, diethylamine, triethylamine, 1-amino-2-propanol-amino-2-(hydroxymethyl)propane-1,3-diol, and 1-(3,4-dihydroxyphenyl)-2 isopropylaminoethanol.

The pharmaceutical will typically be one which is suitable for inhalation and may be provided in any suitable form for this purpose, for example as a solution or powder suspension in a solvent or carrier liquid, for example ethanol, or isopropyl alcohol. Typical propellants are HFA134a, HFA227 and di-methyl ether.

The pharmaceutical may, for example, be one which is suitable for the treatment of asthma. Examples include salbutamol, beclomethasone, salmeterol, fluticasone, formoterol, terbutaline, sodium chromoglycate, budesonide and flunisolide, and physiologically acceptable salts (for example salbutamol sulphate, salmeterol xinafoate, fluticasone propionate, beclomethasone dipropionate, and terbutaline sulphate), solvates and esters, including combinations of two or more thereof. Individual isomers such as, for example, R-salbutamol, may also be used. As will be appreciated, the pharmaceutical may comprise of one or more active ingredients, an example of which is flutiform, and may optionally be provided together with a suitable carrier, for example a liquid carrier. One or more surfactants may be included if desired.

The invention also provides a dispensing apparatus, as described above, in addition, comprising a housing containing a dosage counter comprising at least two annular members and a cog, each mounted rotationally, the housing being suitable for receiving, in use, a container containing a product for dispensation, whereupon each actuation of the apparatus causes the first annular member to incrementally rotate which, after a predetermined number of actuations of the apparatus, causes the cog to rotate, the rotation of the cog causing the second annular member to incrementally rotate.

In use, a longitudinal actuation force applied to the container is converted to a rotational force applied to the first annular member by way of co-operating formations having angled abutment surfaces.

Preferably, the first annular member is provided with angled abutment surfaces.

The dispensing apparatus further comprises a cap or sleeve, for surrounding at least part of a container locatable in the dispensing apparatus. The cap or sleeve is provided with angled abutment surfaces.

Most preferably, the first annular member, and/or the cap or sleeve is/are provided with one or more sets of angled abutment surfaces at different diameters.

In use, respective sets of angled abutment surfaces of the first annular member, and the cap or sleeve co-operate to provide an incremental rotation of the first annular member upon a longitudinal actuation of the sleeve or cap. In particular, a first set of co-operating portions of the first annular member, and the sleeve or cap co-operate to provide a partial-incremental rotation of the annular member upon a down-stroke longitudinal actuation of the dispensing apparatus. A second set of co-operating portions of the first annular member, and the sleeve or cap co-operate to provide a second and final partial-incremental rotation of the first annular member upon a reciprocal up-stroke longitudinal actuation of the dispensing apparatus.

The first annular member may be provided with means for affecting rotation of the cog, such as a notch or protrusion positioned on an outer edge of the first annular member. The notch or protrusion is capable of interacting with a correspondingly-shaped portion of the cog.

When the rotation means of the first annular member is a notch, the cog is turned by way of a tooth catching the notch of the first annular member as the member rotates upon actuation of the apparatus. Similarly, when the rotation means of the first annular member is a protrusion, the cog has a correspondingly-shaped notch with which to affect the desired rotation of the cog.

The first annular member has three rotation means positioned on its outer edge, at least ten increments of rotation apart.

Preferably, each respective part of the cog which interacts with the first and second annular member has a different number of teeth. More preferably, the ratio of teeth on respective parts of the cog which interact with the first and second annular member is 1:2.

The axis of rotation of the cog may be positioned offset from the axes of rotation of both the first and second annular members.

The cog may be made of resilient material and/or resiliently positioned against an outer edge of the first annular member.

Preferably, the first and second annular members are provided with one or more sets of markings indicative of the amount or number of doses of product dispensed from, or remaining in, a container received within the apparatus. In particular, the sets of markings may be numbering or variations of colour and/or tone.

As a preference, numbering on the first annular member ranges from 0 to 9 and that of the second annular member from 00 to 20, so that when both are seen in combination, a three-figure number is shown.

Upon actuation of the apparatus, the numbering is caused to decrease or augment by a value of one.

Preferably, the second annular member is provided with an extended portion which is positioned on the second annular member so as to cover the markings of the first annular member when a container locatable in the housing is empty.

Optionally, the dispensing apparatus may further comprise means for locking-out operation of a container located in the housing.

As a preference, the dispensing apparatus is provided with a mouthpiece which is detachable from the housing.

Additionally, a portion of the sleeve or cap and a portion of the housing may be adapted to provide resistance to actuation of the dispensing apparatus. The portion of the sleeve or cap is a resilient member and the portion of the housing is a static abutment surface which, during use, is temporarily abutable by the portion of the sleeve or cap.

The dispensing apparatus may further comprise third and subsequent annular members, a further cog being provided for each additional annular member, whereby a predetermined number of rotations of the previous annular member causes an incremental rotation of the subsequent member.

The cog of the first aspect comprises a hub, with a pivot hole situated in the centre thereof, and a plurality of teeth outwardly-extending from the centre of the cog, the cog being rotatable about a longitudinal axis through the hole, wherein one or more teeth have a reduced-height in the direction of the longitudinal axis in that upper and lower edges of the tooth are not inline with both upper and lower faces of the cog.

Preferably, the one or more teeth having a reduced height are half the height of other teeth and are interspaced between each pair of non-reduced-height teeth.

Preferably, also, the reduced-height teeth are positioned at either an upper or lower edge of the cog.

Portions of the one or more teeth may be separated by a non-toothed spacer, such that, in use, a first end of the cog, including one or more teeth of reduced height, may interact with the first annular member and a second end of the cog, which is not provided with one or more teeth of reduced height, may interact with the second annular member. The one or more teeth having reduced height are half the height of the non-reduced-height teeth, which non-reduced-height teeth extend from the spacer to the face of the first end of the cog. Preferably, the cog comprises a reduced-height tooth interspaced between each pair of non-reduced-height teeth.

The dispensing apparatus may further comprise a container located in the housing of the dispensing apparatus. The container is, preferably, pressurised. Preferably, the annular members are located around the container. The container is locatable within the dispensing apparatus, such that the container is locatable within the holes/apertures of the at least two annular members. In other words, the annular members surround at least part of the container when the container is loaded within the dispensing apparatus.

The dispensing apparatus may further comprise a container located in the housing of the apparatus.

Preferably, the container located in the apparatus is pressurised.

There is also disclosed a cog comprising a hub, with a pivot hole situated in the centre thereof, and a plurality of teeth outwardly-extending from the centre of the cog, the cog being rotatable about a longitudinal axis through the hole, wherein one or more teeth have a reduced-height in the direction of the longitudinal axis in that upper and lower edges of the tooth are not inline with both upper and lower faces of the cog.

. Preferably, the one or more teeth having a reduced height are half the height of the other teeth and are interspaced between each pair of non-reduced-height teeth.

Preferably, also, the reduced-height teeth are positioned at either an upper or lower edge of the cog and are made of a resilient material.

In order that the invention may be fully disclosed, embodiments will now be described, by way of example only, with reference to the accompanying drawings in which:
Figure 1 is a perspective view of a prior art dispensing apparatus;
Figure 2 is a partial cross-sectional view of a first embodiment of dispensing apparatus, according to the present invention;
Figure 3 is a partial cross-sectional view of the dispensing apparatus of Figure 2;
Figure 4 is a partial cross-sectional view of an embodiment of dispensing apparatus according to the present invention;
Figure 5 is a partial cross-sectional view of the dispensing apparatus of Figure 4;
Figure 6 is a partial cross-sectional view of the dispensing apparatus of Figure 4;
Figure 7 is a partial cross-sectional view of a third embodiment of dispensing apparatus, according to the present invention,
Figure 8 is a partial cross-sectional view of the dispensing apparatus of Figure 7;
Figure 9 is a partial cross-sectional view of a fourth dispensing apparatus, provided by way of example only;
Figure 10 is a partial cross-sectional view of the dispensing apparatus of Figure 9;
Figure 11 is a partial cross-sectional view of a fifth dispensing apparatus, provided by way of example only;
Figure 12 is a partial cross-sectional view of the dispensing apparatus of Figure 11;
Figure 13 is a perspective view of an alternative embodiment of dispensing apparatus according to the present invention;
Figure 14 is an exploded view of the dispensing apparatus of Figure 13, together with a container;
Figure 15 is a perspective view of various internal features of the dispensing apparatus of Figure 13;
Figure 16 is a perspective view of a cog for use in the dispensing apparatus of Figure 13;
Figure 17 is a perspective view of a sleeve for use in the dispensing apparatus of Figure 13;
Figure 18 is a perspective view of first and second number rings and a cog of the dispensing apparatus of Figure 13;
Figure 19 is a partial-cross-sectional view of the dispensing apparatus of Figure 13 and shows internal features of the body of the dispensing apparatus; and
Figure 20 is a perspective view of a first number ring having two different diameter portions, for use with the dispensing apparatus of Figure 13.

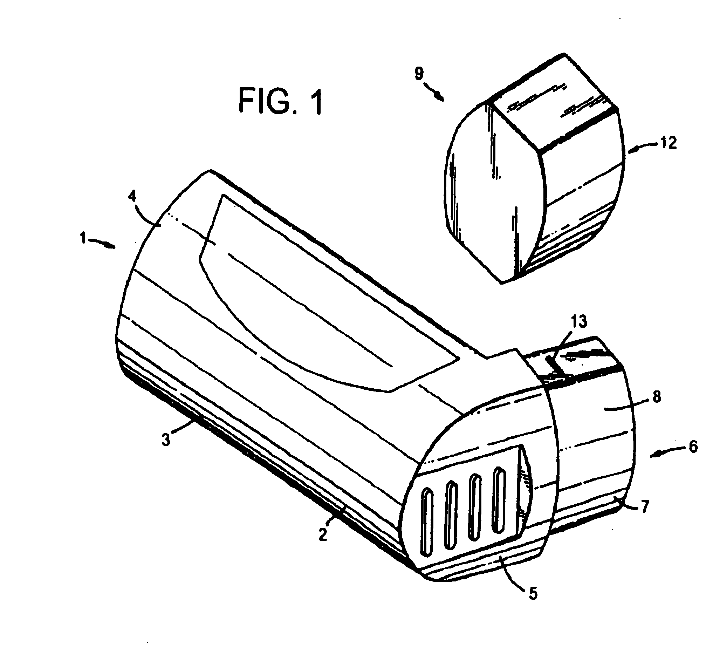

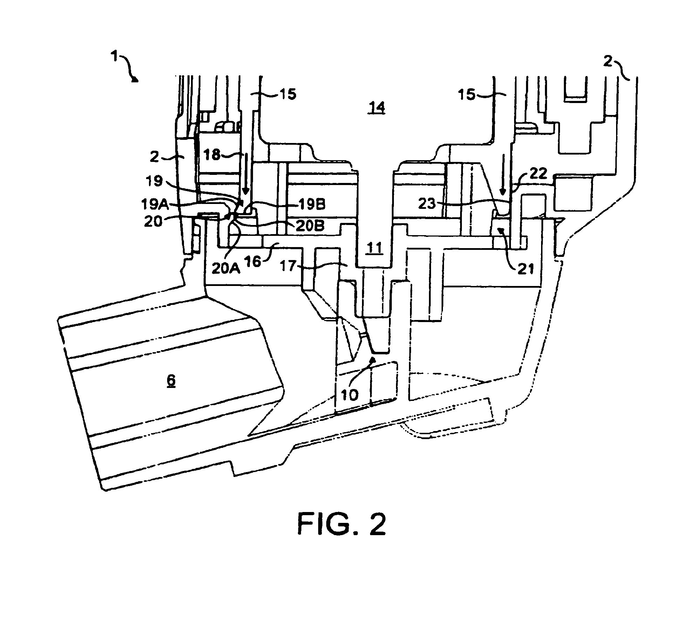

Figure 1 shows a prior art aerosol dispensing apparatus, indicated generally by reference 1. The features shown in Figure 1 are common to all examples of dispensing apparatus 1 described in relation to Figures 2 to 12. The dispensing apparatus 1 is provided with a housing 2 consisting of a tubular body 3 having a tubular side wall and an open end 4. The body 3 is closed at its opposite end by an end wall 5 and a tubular mouthpiece 6 (outlet) projects laterally of the body 3 at a location immediately adjacent the end wall 5. The mouthpiece 6 has a tubular lip portion 7 having an external surface 8 which in use is presented to the lips of a user wishing to inhale orally, via the mouthpiece, an aerosol spray generated from the pressurised dispensing container (not shown) normally received in the body 3. The apparatus 1 further comprises a cap 9. The cap 9 and lip portion 7 are provided with a sliding-fit arrangement such that an internal surface 12 of the cap 9 totally overlays the external surface 8 of the lip portion 7 when the cap is moved into an engaged position in which it is engaged with the mouthpiece 6. The lip portion 7 and the cap 9 are provided with snap-fit connectors which include a detent 13 protruding from the lip portion in co-operating relationship with the groove (not shown) formed in the internal surface 12 of the cap 9. The housing 2 and cap 9 may be formed from plastics material, such as, polypropylene or high-density polyethylene.

Figures 2 to 12 also include the following common features. Preferably, the mouthpiece 6 is detachable. Although the mouthpiece 6 may be connected to the body 3 by any suitable connection means, a bayonet-type connection is preferred. The mouthpiece 6 is provided with a valve stem receiving block 10 (outlet member), having an inlet and an exit passage through which the contents of the container 14 passes following actuation of the dispensing apparatus. Advantageously, the housing 2 includes a base piece 16 which, together, the housing 2 and base piece 16 substantially-fully enclose the dispensing end of the container 14. In particular, access to the valve stem 11 is substantially prevented by the base piece 16 and it substantially prevents a user (or otherwise) tampering with the valve stem 11 and/or actuating the container 14 when the mouthpiece 6 has been removed. Further, the base piece 16 is provided with a hollow joining portion 17 through which product from the container 14 passes when the dispensing apparatus 1 has been actuated. An upper end of the joining portion interacts with the valve stem 11 and a lower end of the joining portion interacts with the inlet of the valve stem receiving block 10.

Preferably, the pressurised dispensing container 14 includes a metering chamber, so as to be able to expel a metered dose of medicament when the dispensing apparatus 1 is actuated. Pressurised dispensing containers 14 are well-known in the art and, typically, product is dispensed from the container 14 following depression of the valve stem against an internal spring. Surrounding at least part of the container 14 and/or the metering chamber is a sleeve 15 (carriage 15). The carriage 15 is provided to receive the container and grip it firmly. The container and carriage 15 can move longitudinally / reciprocally within the body 3 /housing 2 - in response to actuation of the dispensing apparatus - between rest and actuated positions. During periods of non-use, the dispensing apparatus 1 resides in its rest position. Only following application of an actuation force does the container 14 / carriage 15 move downwardly within the housing to provide dispensation of product from the container 14.

The housing 2 further comprises a cover piece 27 (not shown) which is located at the end 4 of the housing 2 and provides a means of closure of the housing 2 at that end. In addition, the cover piece 27 can be removed from the housing 2 to provide access to the inside of the housing 2. Thus, a container 14 may loaded or unloaded from the housing 2 by removing the cover piece 27 and insertion or extraction of the container in or from the carriage 15 - located within the housing. The carriage 15 provides a firm grip on a portion of the container 14 and aids retention of the container 14 within the housing 2. Further, the cover piece 27 may provide - in addition to the carriage 15 - further retention of the container 15 within the housing 2. The cover-piece 27 is, preferably, shaped to receive a non-dispensing end of the container 14, such that, pressing of the cover piece 27 provides movement of the container 14 and carriage 15.

Advantageously, the use of system which requires a minimum force provides less likelihood of partial actuation and/or partial doses of product being dispensed from the container 14.

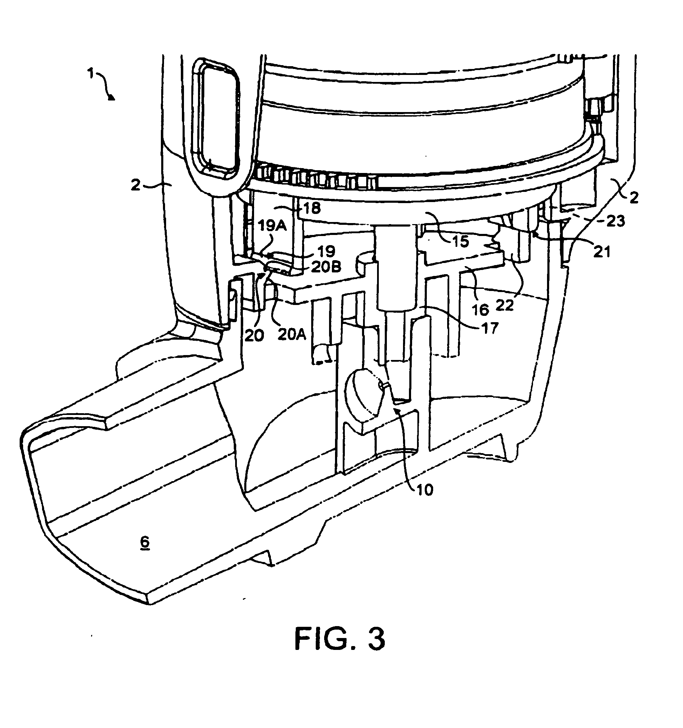

Figures 2 and 3 show a first dispensing apparatus according to the present invention. The carriage 15 is provided with a flexible member 18 on a portion thereof (body of the carriage 15). The flexible member is provided with a projection 19. The flexible member 18 comprises a downwardly dependant arm that extends downwardly towards an abutment 20 located on an inner surface of the housing 2, such that the projection 19 is located on an end of the flexible member 18 remote from the carriage 15. The carriage 15, as mentioned previously, is reciprocally movable which allows the projection 19 to contact the abutment 20 - when the dispensing apparatus is actuated. The projection 19 and abutment 20 are provided to initially resist movement of the container 14 / carriage 15 from a rest position to an actuated position of the container 14. The flexible member 18 is capable of flexing and, during actuation of the dispensing apparatus 1, the flexible member 18 is capable of flexing aufficiently to disengage the projection 19 from the abutment 20 following an actuation force of greater than a predetermined threshold. This predetermined actuation force can be set to any desired level. In a preferred example, the projection 19 and abutment 20 are provided with an opposed inwardly-directed ledge or feature comprising a relatively flat face 19B,20B and an angled face forming an angled trailing face 19A,20A. The flat faces 19B,20B contact one another during actuation of the dispensing apparatus 1. The trailing faces 19A,20A, contact one another as the container 14 returns from the actuated position to the rest position. The trailing faces 19A,20A are angled relative to the direction of movement of the container to allow free and consistent movement of the container back into its rest position, under the action alone of the internal spring force of the container 14. In particular, it will be understood that the arrangement of trailing faces 19A,20A allows the projection 19 and abutment 20 to easily slip past one another following removal of the actuation force on the dispensing apparatus 1.

The housing 2 further comprises guide means 21 for maintaining alignment of the carriage 15 during its reciprocal movement. The guide means 21 is provided by a wall portion 22 of the housing, which makes face-to-face sliding contact with a face 23 of the carriage 15, so as to prevent lateral movement of the carriage 15 during its reciprocal movement. In particular, the guide means 21 maintain correct contact between the projection 19 and the abutment 20, so as to provide correct operation thereof.

In a preferred arrangement, the wall portion 22 of the housing 2 contacts the face 23 of the carriage 15 at a point substantially diametrically opposite the flexible member 18 of the carriage 15.

In use, an actuation force must be applied to the dispensing apparatus 1. The actuation force must be greater than the resistance provided by the projection 19 interacting with the abutment 20 to drive the projection 19 of the flexible member 18 past the abutment 20. As mentioned previously, the actuation force is a predetermined amount of force that can be set to any desired level. The actuation force is applied to the container, typically, through pressing of the cover piece 27. The container 14 and carriage 15 - which are firmly connected - move downwardly, from a rest position of the container 14, bringing the projection 19 of the flexible member 18 in to contact with the abutment 20. There may be a momentary pause until a sufficient force (an actuation force) is applied, which drives the projection 19 past the abutment 20, to the actuated position of the container, and allows depression of the valve stem 11 into the container 14 - which container subsequently dispenses product. Upon removal of the actuation force, the trailing faces 19A, 20A provide for easy movement and slippage of the flexible member 18 and, therefore, the container 14 and carriage 15, upwardly past the abutment 20. The container 14 is then returned to its rest position for further actuation to occur.

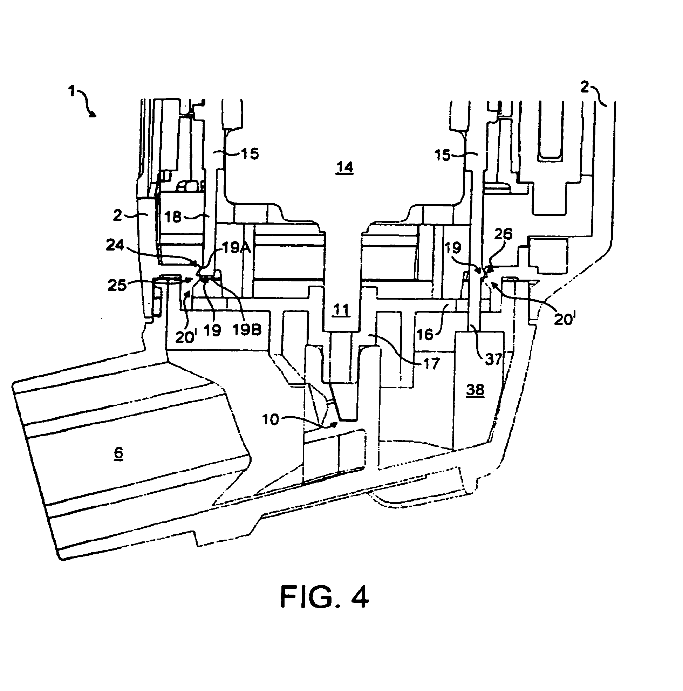

Figures 4 to 6 show a second dispensing apparatus according to the present invention. Like references have been used to indicate common features which have previously been described, and shall not be described further in detail.

As can be seen from Figures 4 to 6, the carriage 15 is provided with a flexible member 18, that has a projection 19. The projection 19 is provided with a flat face 19B and a trailing face 19A, as described in relation to Figures 2 and 3. An abutment 20' is provided having an upper abutment 24 and a lower abutment 25, which together define a carriage retention position. The upper and lower abutments 24,25 are substantially identical in size and shape and are each provided with a flat face 20B and a trailing face 20A. In an alternative, the lower abutment may be slightly smaller in size to provide less resistance to actuation of the dispensing apparatus, following movement of the projection 19 past the upper abutment 24. In particular, the upper and lower abutments 24,25 are spaced apart to provide a space 26 shaped and sized to receive and retain the projection 19 of the carriage 15. It is desirable, for example, during storage or transport of the dispensing apparatus 1, that the container 14 and/or the carriage 15 are immobilised to prevent inadvertent actuation of the dispensing apparatus 1. The abutment 20' provides retention of the carriage whilst the projection 19 is located in the space 26 of the abutment 20'. Naturally, the carriage may be immobilised when received by a user; however, after a first actuation force, the carriage will be free to reciprocally move, as described above in relation to Figures 2 and 3.

This example also includes guide means, as described in relation to the first example.

In an alternative, the dispensing apparatus may be provided with further sets of flexible member 18 and abutments 20', for example, located on a directly opposite side of the dispensing apparatus 1.

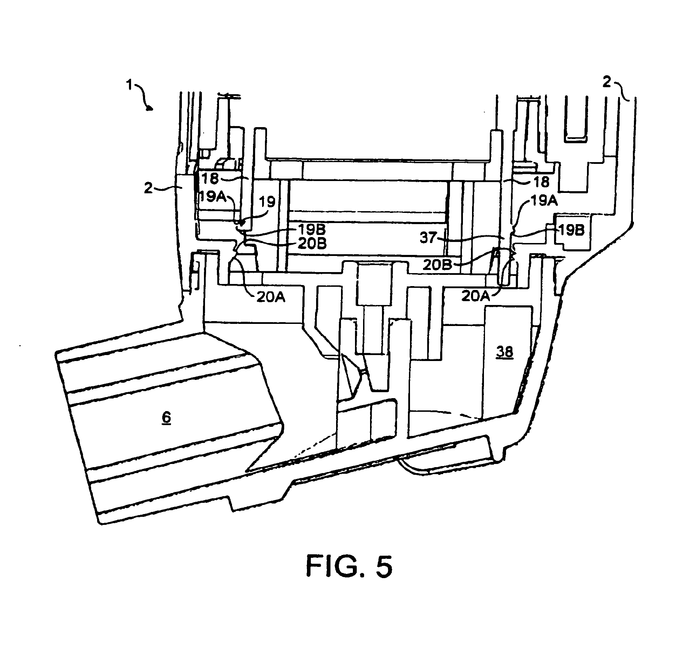

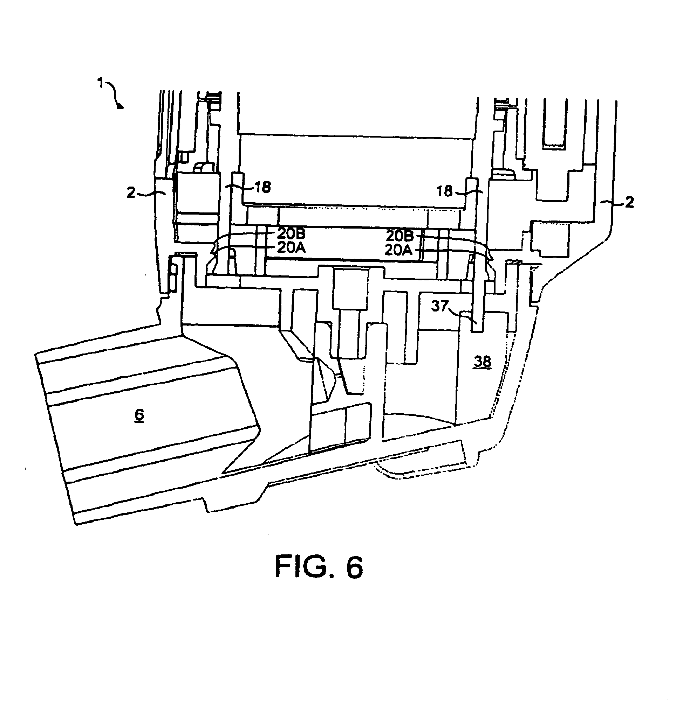

Figure 5 shows, in particular, a dispensing apparatus in its rest position, whereas, Figure 6 shows the dispensing apparatus in its actuated position.

Accordingly, to actuate the dispensing apparatus 1, an actuation force must be applied that is sufficient to drive the projection 19 past both upper and lower abutments 24,25. In every other aspect of actuation of this second example, the features interact as they do for actuation of the first example.

In an alternative example, the space 26 of the abutment 20' defines the rest position of the carriage 15. Accordingly, only the lower abutment 25 must be passed by the projection 19 to obtain actuation of the dispensing apparatus 1, and returned past during the reciprocal movement of the carriage 15. The upper abutment 24 may be formed as a non-return abutment 24 which prevents removal of the carriage 15 and/or the container 14 from the housing 2. For example, a dispensing apparatus may be supplied having the projection 19 located above the upper abutment 24 and, following insertion of a container 14 or initial actuation, for example, the projection 19 is driven past the upper abutment 24 into the space 26. Accordingly, the projection 19 is then prevented from returning to a position above the upper abutment 24 but is not restricted - following actuation - from reciprocally moving below the lower abutment 25 and returning to the space 26.

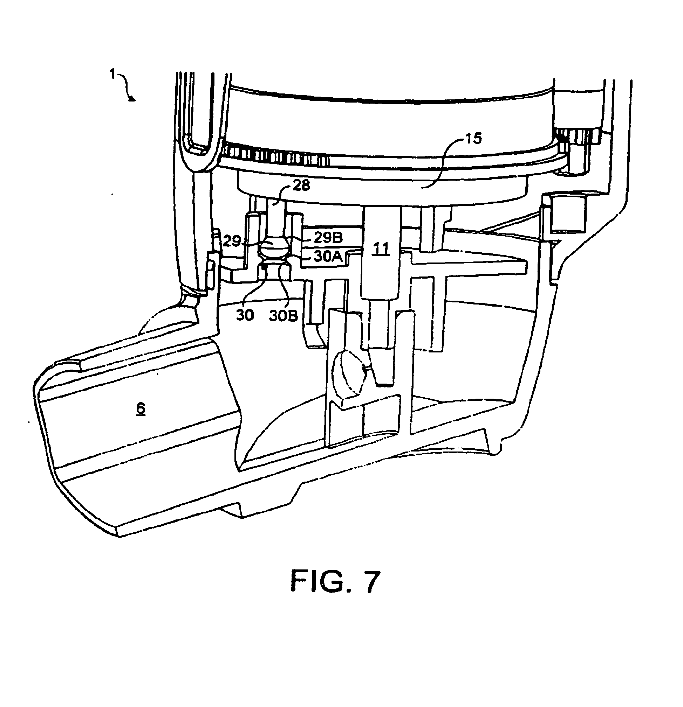

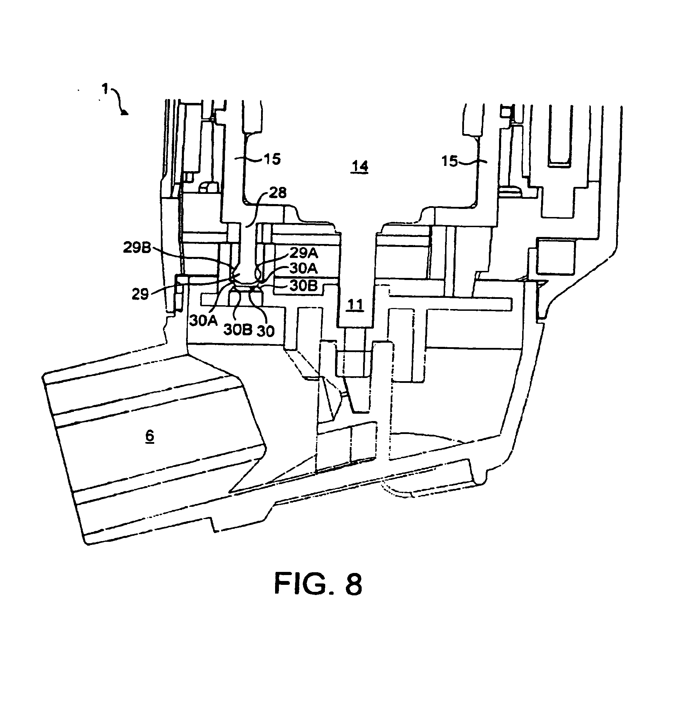

Figures 7 and 8 show a third dispensing apparatus according to the present invention. Like references have been used to indicate common features which have previously been described, and shall not be described further in detail.

In this example, the carriage 15 is provided with an extension member 28 having a ball portion 29. The ball portion 29 is provided on an end of the extension member 28 remote from the carriage 15 and is located so as to interact with and/or engage a socket 30 provided on an internal surface of the housing 2, when the dispensing apparatus 1 is caused to move from a rest position to an actuated position. The socket 30 is provided with an entrance portion 30A, which is shaped and sized to interact with the ball portion 29 and, in particular, a lower portion 29A of the ball portion 29. The size and shape of the entrance portion 30A and/or ball portion 29 / lower ball portion 29A is/are intended to initially resist movement of the container in to the actuated position. Further, the socket 30 is provided with flexure characteristics which allow engagement of the ball portion 29 into the socket 30 when the actuating force reaches a desired level, to allow the container to move into the actuated position. The ball portion 29 and the socket 30 are each provided with trailing faces 29B, 30B which contact one another as the container 14 returns from the actuated position to the rest position. In particular, the trailing faces 29A,30A are shaped and/or angled relative to the direction of movement of the container 14 to allow free and consistent movement of the container 14 back into the rest position under the action alone of the internal spring force of the container 14. As can be seen from Figures 7 and 8, the lower portion 29A and the trailing face 29B, and the entrance portion 30A and the trailing face 30B are differently shaped. Accordingly, owing to the more rounded shape of the lower portion 29A and the entrance portion 30A, it is harder to push the ball portion 29 into the socket 30 to actuate the dispensing apparatus. However, owing to the flatter angled shape of the trailing faces 29A,30A, it is easier for the ball portion 29 to disengage from the socket 30, following removal of the actuation force.

Advantageously, the entrance portion 30A acts as a guide member for maintaining alignment of the carriage 15 during its reciprocal movement within the housing 2.

Accordingly, to actuate the dispensing apparatus 1, an actuation force must be applied that is sufficient to engage the ball portion 29 in the socket 30. From a user's perspective, operation of the dispensing apparatus 1 of this example is identical to operation of the dispensing apparatus of the first two examples.

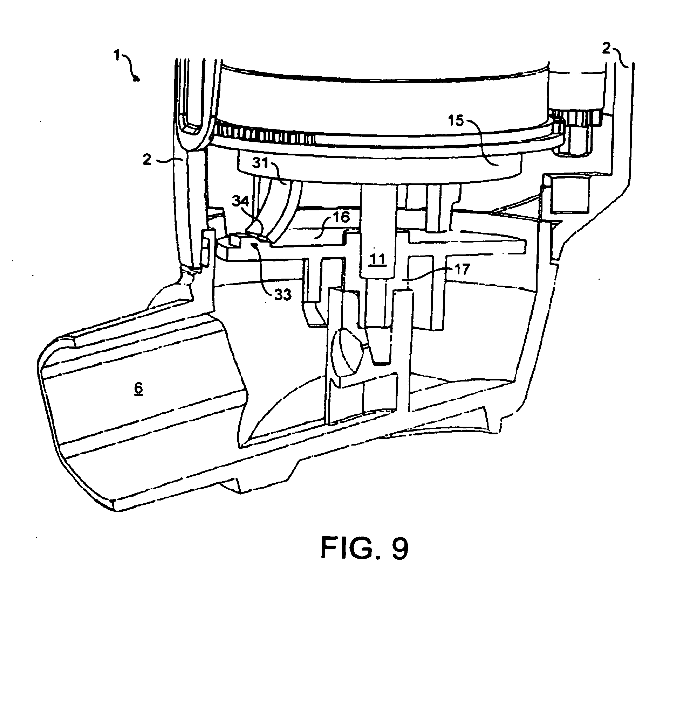

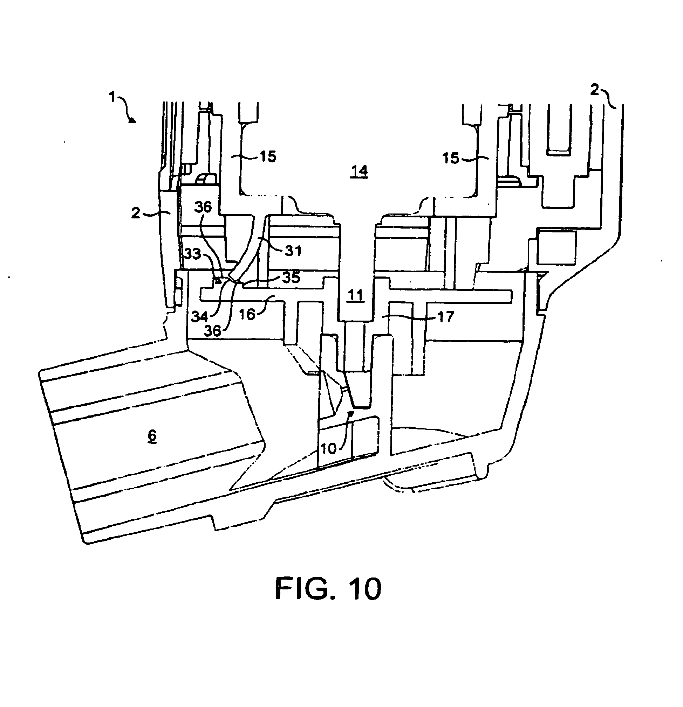

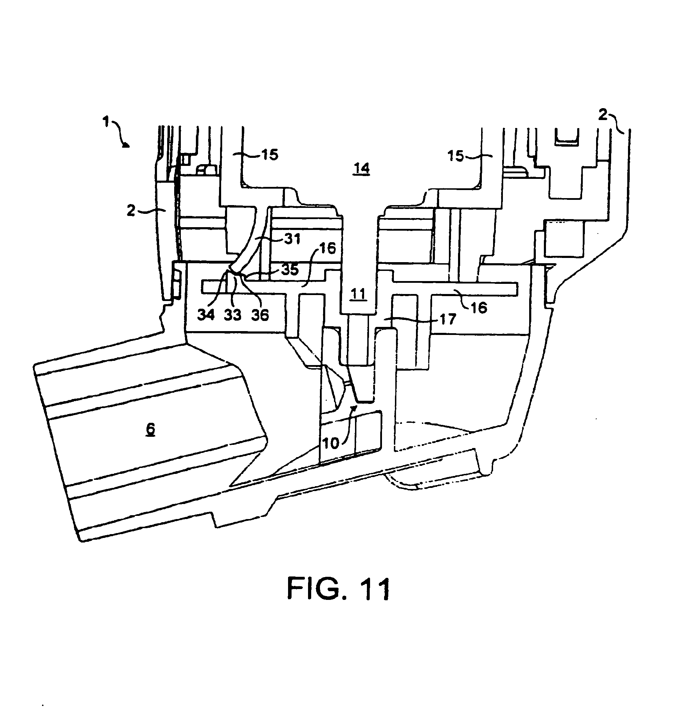

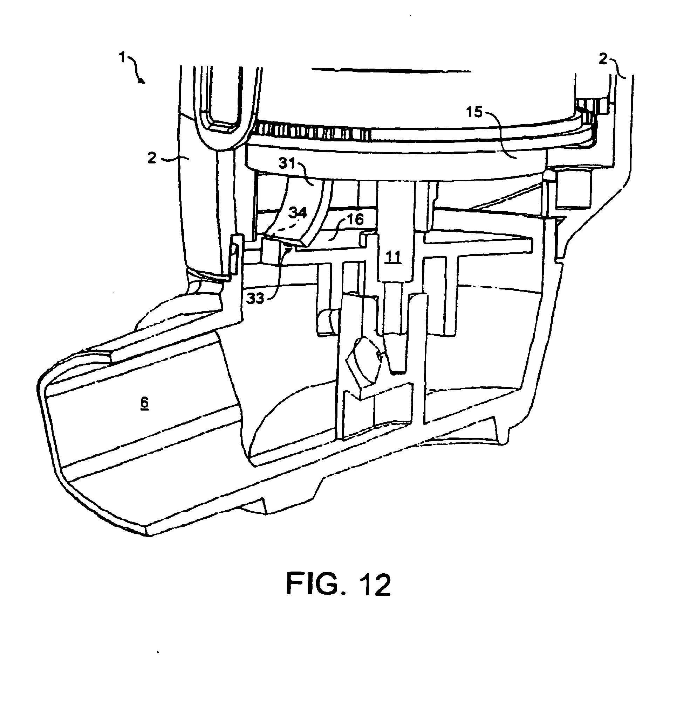

Figures 9 and 10 show a fourth dispensing apparatus. In addition, Figures 11 and 12 show a fifth example. As the fourth and fifth examples are substantially similar, they will be described together. Further, like references have been used to indicate common features which have previously been described, and shall not be described further in detail.

From Figures 9 to 12 it can bee seen that the carriage 15 is provided with a flexible member 31. The flexible member 31 is located on a lower surface 32 of the carriage 15, but in an alternative, may be attached to a side portion of the carriage (not shown). The flexible member 31 is capable of interacting with an abutment 33 provided on an internal surface of the housing 2 but, in particular, on the base piece 16 of the housing 2, when the dispensing apparatus 1 is actuated from a rest position to an actuated position. The interaction of the flexible member 31 and the abutment 33 provides initial resistance to movement of the container in to its actuated position. Advantageously, the flexure characteristics of the flexible member 31 allow it to flex and ride over the abutment 33 when the actuation force reaches a desired level, which allows further movement of the container 14 and carriage 15 to provide dispensation of product from the container 14.

The abutment 33 comprises an obstruction oriented substantially perpendicularly to the direction of reciprocal movement of the container and the abutment 33 comprises an upper profile having one or more ramped surfaces 34. The upper profile of the abutment 33 is also provided with one or more vertical steps 35. In particular, in use, the flexible member 31 and one of the vertical steps 35 initially resists movement of the container 14 into the actuated position until a threshold actuation force is reached, which pushes the flexible member 31, which consequently flexes, and rides over the vertical step and/or at least part of the one or more ramped surfaces 34.

The ramped surfaces 34 are angled relative to the direction of movement of the container 14 to help bias the container 14 back into the rest position, following removal of the actuation force. For example, the resilience of the flexible member 31 acting on the ramped surfaces 34 aids return of the container 14 to its rest position.

Operation of the dispensing apparatus 1 of the fourth and fifth examples is substantially similar to operation of the dispensing apparatus 1 of the first example.

Figures 9 and 10 show an example having an abutment 33 with two horizontal surfaces 36 and Figures 11 and 12 show an example having one horizontal surface. The horizontal surfaces 36 provide regions of reduced resistance to movement of the flexible member 31 and, therefore, provide less resistance to actuation of the dispensing apparatus 1.

A lock-out mechanism may be provided which acts between the carriage 15 and a portion of the housing 2, to prevent inadvertent actuation of the dispensing apparatus 1. Aspects of the lock-out mechanism can be seen in Figures 4 to 6. The housing comprises upper and lower portions. In one example, the upper portion is the housing 2 and the lower portion is the detachable mouthpiece 6. The upper portion 2 (housing 2) houses at least the container 14. The lower portion 6 (mouthpiece 6) comprises the outlet. The upper and lower portions are conjoined, with a bayonet-type fixture for example, and may rotate between relative locked and unlocked positions. In particular, the carriage 15 is provided with a projection 37 that extends downwardly beyond the remainder of the carriage 15 towards the outlet end 6 or mouthpiece 6. An abutment 38 is located in the detachable mouthpiece 6. In the locked position, the abutment 38 is aligned with the carriage projection 37 so as to prevent movement of the carriage 15 into the actuated position of the container 14 and, in the unlocked position, the abutment 38 is out of alignment with the carriage projection 37 so as to allow movement of the carriage 37 into the actuated position. Further reciprocal movement of the carriage 15 is allowed.

The lower part 6 rotates about the longitudinal axis of the upper part 2 between locked and unlocked positions.

The projection 37 may form an extension part of one of the flexible members 18, as shown in Figures 4 to 6.

Advantageously, the lock-out mechanism aids insertion or extraction of a container 14 from the housing 2 by immobilising the carriage 15.

Advantageously, the pre-compression features discussed above may be utilised with a dispensing apparatus having a mechanical dose counter.

It will be appreciated that the lock-out mechanism can be applied to a variety of dispensing apparatus, including, at least, any of the above-disclosed examples.

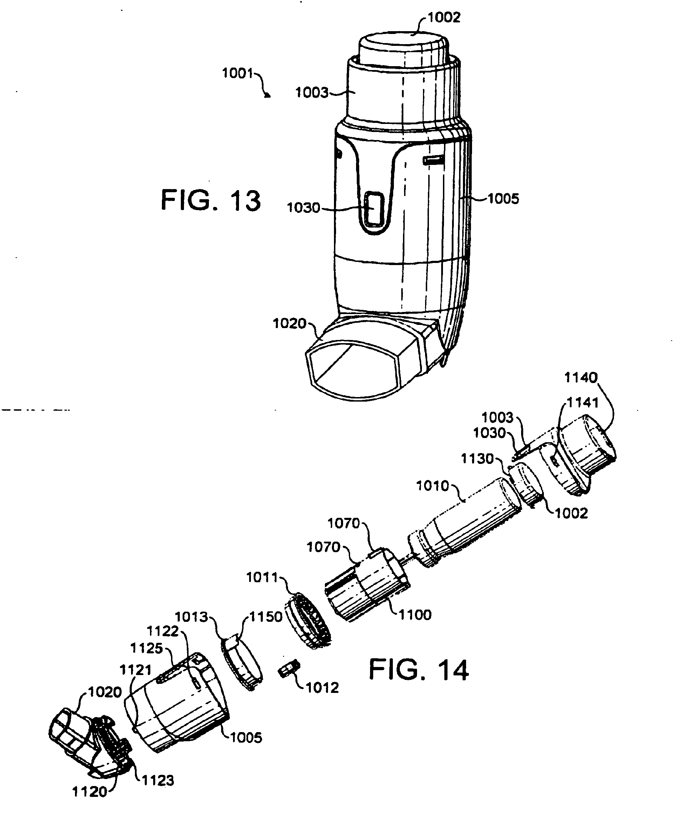

Figures 13 to 20 show another dispensing apparatus which is fully described in the applicants co-pending application PCT/GB2005/002007. Additional features of this apparatus may optionally and advantageously be incorporated in any of the dispensing apparatus described above as will be described below by way of example.

The apparatus is indicated generally at 1001 and comprises a mechanism for counting doses as they are dispensed from a container contained within the apparatus. The dispensing apparatus 1001 has a cap 1002, fixing collar 1003, a main body 1005 and a detachable mouthpiece 1020. The dispensing apparatus is also provided with first and second number rings 1011,1013, a cog 1012 and a sleeve 1100, as shown in Figure 14.

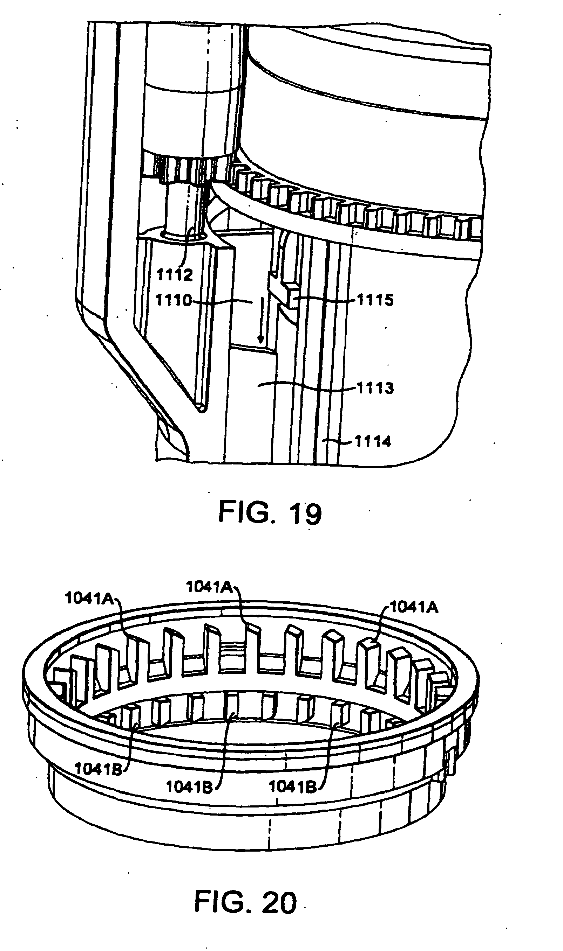

In this apparatus, only one body portion (main body 1005) is provided. The main body 1005 houses the number rings 1011,1013, cog 1012 and container 1010 on assembly. As shown in more detail in Figure 19, the number rings 1011,1013 may rest upon internal projections 1110 of the main body 1005. Such internal projections 1110 provide up-facing surfaces 1111 upon which the second number ring 1013 may rest and rotate, during use. The first number ring 1011 rests and rotates, during use, on top of the second number ring 1013. The cog 1012 is rotatably mounted within the main body 1005 on a cylindrical portion 1112 and interacts with both first and second number rings 1011,1013. The internal projections 1110, of which there are typically four, also interact with the sleeve 1100. In the illustrated apparatus the main body 1005 is provided with a static abutment surface 1113 which may interact with at least part of the sleeve 1100.

The main body 1005 is also provided with a hollow elongate portion 1121 into which the valve stem 1022 of a loaded container 1010 passes. This elongate portion 1121 provides a seal with the valve stem 1022 and an abutment against which the valve stem is pressed during actuation of the dispensing apparatus. The elongate portion is arranged to co-operate with a correspondingly-shaped portion of the mouthpiece 1020. The elongate portion 1121 provides protection for the valve stem when the mouthpiece 1020 has been removed. In addition, the main body 1005 is provided with one or more slots 1122 in an upper region for interaction with corresponding parts of the fixing collar 1003.

The detachable mouthpiece 1020 is provided with one or more fixtures 1120 for attaching the mouthpiece 1020 to the main body 1005. In particular, a push-fit attachment is provided by the mouthpiece 1020 having one or more internal armatures 1120 each comprising a notch 1123 which cooperates with a corresponding groove (not shown) located in the internal surface of the main body 1005 on assembly. Accordingly, it is very simple to change the mouthpiece of the dispensing apparatus, if desired. The mouthpiece 1020 is also provided with a block (not shown) for receipt of the valve stem 1022 of a loaded container 1010 or the elongate portion 1121. A dust cap (not shown) may be placed over the open distal end of the mouthpiece 1020.

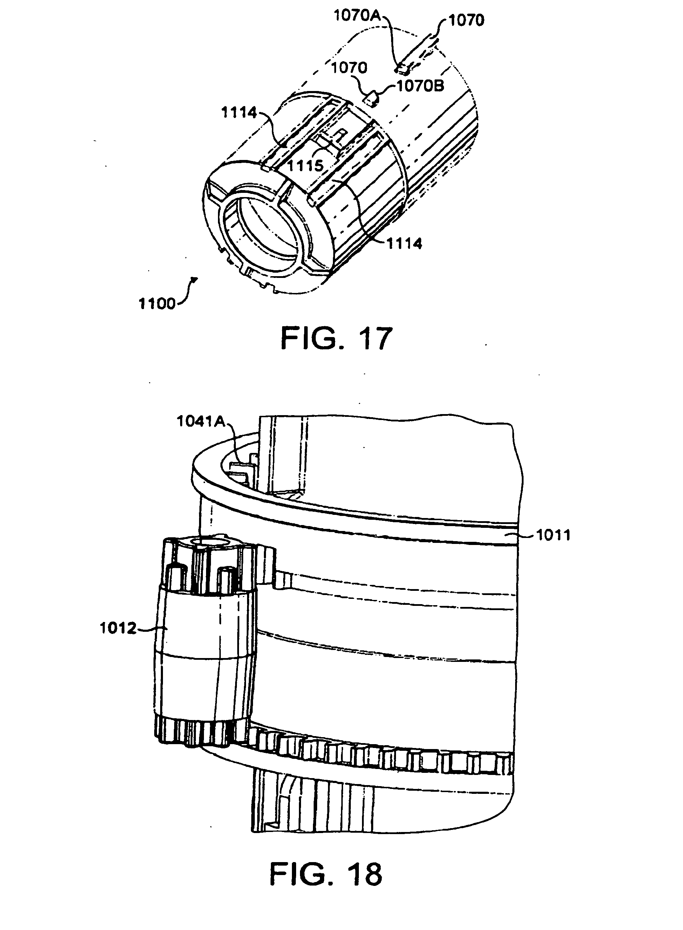

As shown in Figures 14 and 17, the sleeve 1100 comprises an open-ended cylinder having an upper end which can receive a container 1010 to be located in the dispensing apparatus 1001 and a lower end which has a reduced diameter opening through which the valve stem 1022 of a container 1010, located within the sleeve 1100 may protrude from but through which the body of the container 1010 cannot pass. The sleeve 1100 is further provided with a number of projections 1070 on its exterior having angled abutment surfaces 1070a,1070b for co-operation with angled abutment surfaces 1041 of the first number ring 1011, as described below. Preferably, two sets of two projections 1070 are provided on opposite outsides of the sleeve 1100. Each set comprises two spaced-apart opposing angled abutment surfaces 1070a, 1070b which are angled in a manner as mirror images of each other. The two sets are oriented in the same direction to provide only one direction of rotation of the number ring 1011. Additionally, the sleeve 1100 is provided with slots 1114 into which the internal projections 1110 of the main body 105 can slide during location of the sleeve 1100, within the main body 1005 and actuation of the dispensing apparatus. The sleeve 1100 is also provided with a resilient member 1115, in the form of a sprung cantilevered catch, for interaction with the static abutment surface 1113 of the main body 1005, as will be described below.

As shown in Figure 14, the cap 1002 fits over the upper end of the container 1010, opposite the metering valve. The cap 1002 is provided with a plurality of external flange portions 1130, for interaction with the fixing collar 1003.

The fixing collar 1003 is provided with one or more notches 1141 for locating in the one or more slots 1122 of the main body 1005, so as to provide an improved push-fit attachment of the fixing collar 1003 to the main body 1005. The fixing collar 1003 is provided with internal flange portions 1140, such that, the external flange portions 1130 of the cap 1002 interact with the internal flange portions 1140 to retain the cap 1002 and, therefore, the container 1010, within the main body 1005 when closed. The fixing collar 1003 is provided with a clear portion 1030, or one or more apertures 1030 through which portions provided with markings of number rings 1011, 1013 are visible. Preferably, the fixing collar 1003 is provided with a projection 1124 at the end of which is provided the clear portion 1030 or one or more apertures 1030. This projection 1124 extends into the region of the main body 1005 into a correspondingly-shaped hole 1125. This arrangement provides the clear portion 1030 or the one or more apertures 1030 at a position in the region of the main body 1005 which allows viewing of the markings on the number rings 1011, 1013.

The fixing collar 1003, in combination with the cap 1002, provides a closure to the upper end of the main body 1005, remote from the mouthpiece 1020. In addition, the internal parts of the dispensing apparatus, for example, the number rings 1011, 1013, the cog 1012, the sleeve 1100, and the container are held within the main body 1005 by the fixing collar 1003 and the cap 1002.

The first number ring 1011 may be provided with two rows of angled abutment surfaces 1041 located on two different diameter portions of that number ring. The angled abutment surfaces 1041 of a larger diameter portion are indicated by reference 1041a and those of a smaller diameter by reference 1041b, as shown in Figure 20, in particular.

The second number ring 1013 is provided with an extended portion 1150 which is positioned to enable covering of the markings on the first number ring 1011 when a container locatable in the housing is empty. Advantageously, the extended portion 1150 provides a clear indication to a user that the dispensing apparatus has provided its full-quota of dispensations.

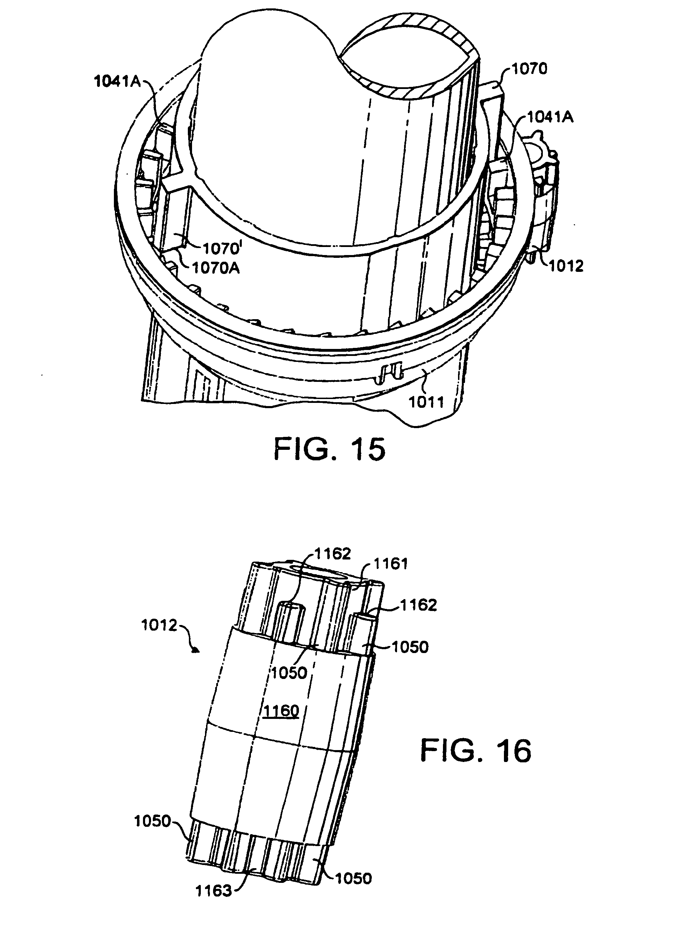

The cog 1012, as shown in Figure 16 in particular, is provided with one or more teeth 1050 separated by a non-toothed spacer 1160, such that, a first end 1161 of the cog 1012, including one or more teeth 1162 of reduced height, may interact with the first annular member 1011 and a second end of the cog 1163, which is not provided with one or more teeth of reduced height, may interact with the second annular member 1013. The one or more teeth 1162 having reduced height are, typically, half the height of the non-reduced-height teeth, which non-reduced-height teeth extend from the spacer 1160 to the face of the first end 1161 of the cog 1012. Most preferably, cog 1012 is provided with a reduced-height tooth 1162 interspaced between each pair of non-reduced-height teeth.

In use, the dispensing apparatus is actuated in a similar manner to the dispensing apparatus described above. Additionally, the slots 1114 of the sleeve 1100 slide down the internal projections 1110 of the main body 1005 during actuation and, thus, guiding the valve stem 1022 against the abutment (not shown) within the elongate portion 1121. In addition, the resilient member 1115 of the sleeve 1100 abuts the static abutment surface 1113 of the main body 1005. In the apparatus as described in PCT/GB2005/002007 such abutment provides increased resistance to actuation above the normal resistance provided by the reaction force of the valve stem 1022 which must be overcome before actuation can occur. It will be appreciated that the mechanisms described above with reference to Figures 2 to 12 will replace the need for abutment surface 1113 and resilient member 1115.

The present arrangement provides partial-incremental rotations of the first number ring 1011. When the dispensing apparatus is actuated - by a longitudinal down-stroke of the cap 1002 and the container 1010 - a first angled abutment surface 1070a of each set of portions 1070 interacts with at least one angled abutment surface 1041a of the larger diameter portion of the first number ring 1011 to provide a partial incremental rotation of the first number ring 1011. Further, during a corresponding reciprocal up-stroke of the cap 1002 and the container 1010, a second angled abutment surface 1070b of each set of portions 1070 interacts with at least one angled abutment surface 1041b of the smaller diameter portion of first number ring 1011 to provide a second and final partial-incremental rotation of the first number ring 1011. Therefore, an augmented or decreased number is indicated to a user by a two-step rotation of the first number ring 1011 upon actuation of the dispensing apparatus 1001.

The number rings 1011, 1013 surround or are located around the container 1001, when the container 1010 is loaded in the dispensing apparatus 1001. Further, the sleeve 1100 and the container 1010 are slidable within the main body 1005 and pass through the holes/apertures of the number rings 1011, 1013.

The dose counter mechanism described above with reference to Figures 13 to 20 represents only one type of counter that may be utilised in combination with the features of Figures 2 to 12. It will be clear to the skilled person that other types of counter, both mechanical and otherwise may alternatively be used.

## Claims

1. Aerosol dispensing apparatus (1) comprising a horsing (2) in which a container (14) can be located, an outlet (6) leading from the housing, a receiving block (10) having an inlet for receiving an outlet member (11) of the container and a passage through which contents of the container may pass on their way to the outlet when the container is actuated, the housing containing a carriage (15) in which the container is firmly gripped, the carriage being able to slide reciprocally within the housing as the container moves between rest and actuated positions, the carriage comprising a flexible member (18; 28) having a projection (19; 29) provided thereon, said projection being located so as to contact an abutment (20; 30) provided on the housing when the container is moved under an actuating force from the rest position towards the actuated position so as to initially resist movement of the container into the actuated position, the flexible member (18, 28) being free to flex sufficiently to disengage the projection from the abutment when the actuating force reaches a desired level to allow the container to move into the actuated position, **characterised by** the projection and the abutment each having trailing faces (19A; 20A; 29B; 30B) which contact one another as the container returns from the actuated position to the rest position, wherein the trailing faces are angled relative to the direction of movement of the container to allow a reduced resistance to movement and consistent movement of the container back into the rest position under the action alone of an internal spring force of the container.

2. Aerosol dispensing apparatus as claimed in claim 1 wherein the housing (2) further comprises guide means (21) for maintaining alignment of the carriage (15) during its reciprocal movement.

3. Aerosol dispensing apparatus as claimed in claim 2 wherein the guide means (2) comprises a wall portion (22) of the housing which makes face-to-face sliding contact with a face (23) of the carriage so as to prevent lateral movement of the carriage during its reciprocal movement.

4. Aerosol dispensing apparatus as claimed in claim 3 wherein the wall portion (22) of the housing contacts the face (13) of the carriage at a point substantially diametrically opposite the flexible member (18) of the carriage.

5. Aerosol dispensing apparatus as claimed in any preceding claim wherein the abutment comprises a single inwardly-directed ledge or feature (20) comprising a relatively flat upper face and an angled lower face forming the angled trailing face (20A).

6. Aerosol dispensing apparatus as claimed in claim 1 wherein the projection comprises a ball portion (29) and the abutment comprises a socket (30).

7. Aerosol dispensing apparatus as claimed in any preceding claim wherein the flexible member (18; 28) comprises a downwardly dependent arm located on a body of the carriage with the projection located at a distal end of the downwardly dependant arm.

8. Aerosol delivery apparatus (1) as claimed in any preceding claim further comprising a dose counter.

9. Aerosol delivery apparatus as claimed in claim 8 wherein the dose counter is mechanical.

10. Aerosol delivery apparatus as claimed in claim 9 wherein actuation of the dose counter to increment or decrement the count is controller by movement of the carriage means (15) into the actuated position of the container.

11. Aerosol delivery apparatus as claimed in any one of claims 8 to 10, further comprising a housing containing a dosage counter comprising at least two annular members (1011; 1013) and a cog (1102), each mounted rotationally, the housing being suitable for deceiving, in use, a container containing a product for dispensation, whereupon each actuation of the apparatus causes the first annular member (1011) to incrementally rotate which, after a predetermined number of actuations of the apparatus, causes the cog (1102) to rotate, the rotation of the cog causing the second annular member (1013) to incrementally rotate.

12. Aerosol delivery apparatus as claimed in any one of claims to 11, wherein the axis of rotation of the cog (1102) is positioned offset from the axes of rotation of both the first and second annular members (1011; 1013).

13. Aerosol delivery apparatus as claimed in any one of claims 8 to 12, wherein the cog (1102) comprises a hub (1160), with a pivot hole situated in the centre thereof, and a plurality of teeth (1050) outwardly-extending from the centre of the cog, the cog being rotatable about a longitudinal axis through the hole, wherein one or more teeth have a reduced height (1162) in the direction of the longitudinal axis, in that upper and lower edges of the tooth are not inline with both upper and lower faces of the cog.

14. Aerosol delivery apparatus as claimed in any one of claims 11 to 13, wherein the first and second annular members (1011; 1013), in use, surround at least part of a container (1030) of product or a metering valve thereof located in the housing.

## Patentansprüche

1. Aerosol-Abgabegerät (1), umfassend ein Gehäuse (2), in welchem ein Behälter (14) angeordnet werden kann, einen Auslass (6), der von dem Gehäuse weg führt, einen Aufnahmeblock (10), der einen Einlass zum Aufnehmen eines Auslassglieds (11) des Behälters aufweist, und einen Durchgang, durch welchen der Inhalt des Behälters auf seinem Weg zu dem Auslass passieren kann, wenn der Behälter betätigt wird, wobei das Gehäuse einen Schlitten (15) enthält, in welchem der Behälter fest gehalten ist, wobei der Schlitten dazu in der Lage ist, innerhalb des Gehäuses hin und her zu gleiten, wenn sich der Behälter zwischen einer Ruhestellung und einer Betriebssteltung bewegt, wobei der Schlitten ein biegsames Glied (18; 28) mit einem darauf vorgesehenen Vorsprung (19; 29) aufweist, wobei der Vorsprung derart angeordnet ist, dass er mit einem an dem Gehäuse vorgesehenen Anschlag (20; 30) in Kontakt tritt, wenn der Behälter unter einer Betätigungskraft von der Ruhestellung zu der Betriebsstellung bewegt wird, um einer Bewegung des Behälters in die Betriebsstellung zunächst entgegenzuwirken, wobei das biegsame Glied (18; 28) frei ist, sich ausreichend zu biegen, um den Vorsprung von dem Anschlag zu lösen, wenn die Betätigungskraft ein gewünschtes Maß erreicht, um dem Behälter zu erlauben, sich in die Betriebsstellung zu bewegen, **dadurch gekennzeichnet, dass** der Vorsprung und der Anschlag jeweils Endflächen (19A; 20A; 29B; 30B) aufweisen, die in Kontakt miteinander treten, wenn der Behälter aus der Betriebsstellung zu der Ruhestellung zurückkehrt, wobei die Endflächen relativ zu der Bewegungsrichtung des Behälters abgewinkelt sind, um einen reduzierten Widerstand gegen eine Bewegung und eine gleichmäßige Bewegung des Behälters zurück in die Ruhestellung zu erlauben, unter der Einwirkung allein einer internen Federkraft des Behälters.

2. Aerosol-Abgabegerät nach Anspruch 1, wobei das Gehäuse (2) weiterhin ein Führungsmittel (21) umfasst, um eine Ausrichtung des Schlittens (15) während seiner Hin- und Her-Bewegung aufrechtzuerhalten.

3. Aerosol-Abgabegerät nach Anspruch 2, wobei das Führungsmittel (2) einen Wandabschnitt (22) des Gehäuses umfasst, welcher einen Gleitkontakt Fläche an Fläche mit einer Fläche (23) des Schlitten herstellt, um eine seitliche Bewegung des Schlitten während seiner Hin- und Her-Bewegung zu verhindern.

4. Aerosol-Abgabegerät nach Anspruch 3, wobei der Wandabschnitt (22) des Gehäuses in einem Punkt im Wesentlichen diametral gegenüber von dem biegsamen Glied (18) des Schlittens in Kontakt mit der Fläche (13) des Schlittens steht/tritt.

5. Aerosol-Abgabegerät nach einem der vorhergehenden Ansprüche, wobei der Anschlag einen einzelnen nach innen gerichteten Vorsprung oder ein Element (20) umfasst, der/das eine relativ flache Oberseite und eine abgewinkelte Unterseite umfasst, welche die abgewinkelte Endfläche (20A) bildet.

6. Aerosol-Abgabegerät nach Anspruch 1, wobei der Vorsprung einen Kugelabschnitt (29) umfasst und der Anschlag eine Buchse (30) umfasst.

7. Aerosol-Abgabegerät nach einem vorhergehenden Anspruch, wobei das flexible Glied (18; 28) einen nach unten herabhängenden Arm umfasst, der sich an einem Körper des Schlittens befindet, wobei der Vorsprung an einem distalen Ende von dem nach unten herabhängenden Arm angeordnet ist.

8. Aerosol-Abgabegerät (1) nach einem vorhergehenden Anspruch, weiter umfassend einen Dosiszähler

9. Aerosol-Abgabegerät nach Anspruch 8, wobei der Dosiszähler mechanisch ist.

10. Aerosol-Abgabegerät nach Anspruch 9, wobei eine Betätigung des Dosiszählers, um den Zählwert zu inkrementieren oder zu dekrementieren, durch eine Bewegung von dem Schlitten-Mittel (15) in die Betriebsstellung des Behälters gesteuert/geregelt wird.

11. Aerosol-Abgabegerät nach einem der Ansprüche 8 bis 10, weiter umfassend ein Gehäuse, welches einen Dosiszähler enthält, der wenigstens zwei ringförmige Glieder (1011; 1013) und eine Zahnung (1102) umfasst, die jeweils drehbar angebracht sind, wobei das Gehäuse dazu geeignet ist, im Betrieb einen Behälter aufzunehmen, der ein Produkt zur Abgabe enthält, wobei jede Betätigung des Geräts bewirkt, dass das erste ringförmige Glied (1011) sich inkrementell dreht, was, nach einer vorbestimmten Anzahl von Betätigungen des Geräts, bewirkt, dass die Zahnung (1102) sich dreht, wobei die Drehung der Zahnung bewirkt, dass sich das zweite ringförmige Glied (1013) inkrementell dreht.

12. Aerosol-Abgabegerät nach einem der Ansprüche 8 bis 11, wobei die Drehachse der Zahnung (1102) in Bezug auf die Drehachsen sowohl von dem ersten als auch von dem zweiten ringförmigen Glied (1011; 1013) versetzt positioniert ist.

13. Aerosol-Abgabegerät nach einem der Ansprüche 8 bis 12, wobei die Zahnung (1102) eine Nabe (1160) umfasst, mit einem Schwenk-Loch, das sich in der Mitte davon befindet, und mit einer Mehrzahl von Zähnen (1050), die sich von der Mitte der Zahnung nach außen erstrecken, wobei die Zahnung um eine Längsachse durch das Loch drehbar ist, wobei ein Zahn oder mehrere Zähne eine reduzierte Höhe (1162) in der Richtung der Längsachse aufweisen, wobei obere und untere Ränder des Zahnes sich nicht mit beiden oberen und unteren Flächen der Zahnung in einer Linie befinden.

14. Aerosol-Abgabegerät nach einem der Ansprüche 11 bis 13, wobei das erste und das zweite ringförmige Glied (1011; 1013) im Betrieb wenigstens einen Teil von einem Produkt-Behälter (1030) oder von einem Dosierventil davon umgeben, der/das in dem Gehäuse angeordnet ist

## Revendications

1. Appareil de distribution d'aérosol (1) comprenant un boîtier (2) dans lequel un récipient (14) peut être placé, une sortie (6) menant hors du boîtier, un bloc de réception (10) ayant une entrée destinée à recevoir un élément de sortie (11) du récipient et un passage à travers lequel le contenu du récipient peut passer sur son chemin vers la sortie lorsque le récipient est actionné, le boîtier contenant un chariot (15) dans lequel le récipient est fermement tenu, le chariot pouvant coulisser en va-et-vient dans le boîtier à mesure que le récipient se déplace entre des positions de repos et d'actionnement, le chariot comprenant un élément flexible (18 ; 28) ayant une saillie (19 ; 29) prévue dessus, ladite saillie étant située de manière à entrer en contact avec une butée (20 ; 30) prévue sur le boîtier lorsque le récipient est déplacé sous l'effet d'une force d'actionnement depuis la position de repos vers la position actionnée de manière à résister au début à un mouvement du récipient dans la position actionnée, l'élément flexible (18 ; 28) pouvant suffisamment fléchir pour désengager la saillie de la butée lorsque la force d'actionnement atteint un niveau souhaité pour permettre au récipient de se déplacer vers la position actionnée, **caractérisé en ce que** la saillie et la butée ayant chacune des faces arrières (19A ; 20A ; 29B ; 30B) qui entrent en contact les unes avec les autres lorsque le récipient retourne de la position actionnée à la position de repos, où les faces arrières sont inclinées par rapport à la direction de mouvement du récipient afin de permettre une résistance réduite au mouvement et un mouvement uniforme de retour du récipient vers la position de repos sous la seule action d'une force de rappel interne du récipient.

2. Appareil de distribution d'aérosol tel que revendiqué dans la revendication 1, dans lequel le boîtier (2) comprend en outre un moyen de guidage (21) pour maintenir un alignement du chariot (15) pendant son mouvement de va-et-vient.

3. Appareil de distribution d'aérosol tel que revendiqué dans la revendication 2, dans lequel le moyen de guidage (2) comprend une partie de paroi (22) du boîtier qui établit un contact direct de coulissement avec une face (23) du chariot de manière à empêcher un mouvement latéral du chariot pendant son mouvement de va-et-vient.

4. Appareil de distribution d'aérosol tel que revendiqué dans la revendication 3, dans lequel la partie de paroi (22) du boîtier entre en contact avec la face (13) du chariot au niveau d'un point essentiellement diamétralement opposé à l'élément flexible (18) du chariot.

5. Appareil de distribution d'aérosol tel que revendiqué dans l'une des revendications précédentes, dans lequel la butée comprend un(e) unique rebord ou forme (20) dirigé(e) vers l'intérieur comprenant une face supérieure relativement plane et une face inférieure inclinée formant la face arrière inclinée (20A).

6. Appareil de distribution d'aérosol tel que revendiqué dans la revendication 1, dans lequel la saillie comprend une partie sphérique (29) et la butée comprend une douille (30).

7. Appareil de distribution d'aérosol tel que revendiqué dans l'une des revendications précédentes, dans lequel l'élément flexible (18 ; 28) comprend un bras se prolongeant vers le bas situé sur un corps du chariot avec la saillie située au niveau d'une extrémité distale du bras se prolongeant vers le bas.

8. Appareil de distribution d'aérosol (1) tel que revendiqué dans l'une des revendications précédentes, comprenant en outre un compteur de doses.

9. Appareil de distribution d'aérosol tel que revendiqué dans la revendication 8 dans lequel le compteur de doses est mécanique.

10. Appareil de distribution d'aérosol tel que revendiqué dans la revendication 9, dans lequel l'actionnement du compteur de doses pour incrémenter ou décrémenter le comptage est commandé par un mouvement du moyen de transport (15) dans la position actionnée du récipient.

11. Appareil de distribution d'aérosol tel que revendiqué dans l'une quelconque des revendications 8 à 10, comprenant en outre un boîtier contenant un compteur de doses comprenant au moins deux éléments annulaires (1011 ; 1013) et un élément à dents (1102), chacun étant monté de manière rotative, le boîtier étant adapté à recevoir, lors de l'utilisation, un récipient contenant un produit à distribuer, à la suite de quoi chaque actionnement de l'appareil entraîne la rotation progressive du premier élément annulaire (1011) qui, après un nombre prédéterminé d'actionnements de l'appareil, entraîne la rotation de l'élément à dents (1102), la rotation de l'élément à dents entraîne la rotation progressive du deuxième élément annulaire (1013).

12. Appareil de distribution d'aérosol tel que revendiqué dans l'une quelconque des revendications 8 à 11, dans lequel l'axe de rotation de l'élément à dents (1102) est positionné de façon à être décalé des axes de rotation à la fois des premier et deuxième éléments annulaires (1011 ; 1013).

13. Appareil de distribution d'aérosol tel que revendiqué dans l'une quelconque des revendications 8 à 12, dans lequel l'élément à dents (1102) comprend un moyeu (1160), avec un trou de pivot situé en son centre, et une pluralité de dents (1050) s'étendant vers l'extérieur à partir du centre de l'élément à dents, l'élément à dents pouvant tourner autour d'un axe longitudinal à travers le trou, où une ou plusieurs dents ont une hauteur réduite (1162) dans la direction de l'axe longitudinal, de sorte que des bords supérieur et inférieur des dents ne sont pas alignés avec les deux faces supérieure et inférieure de l'élément à dents.

14. Appareil de distribution d'aérosol tel que revendiqué dans l'une quelconque des revendications 11 à 13, dans lequel les premier et deuxième éléments annulaires (1011 ; 1013), lors de l'utilisation, entourent au moins une partie d'un récipient (1030) de produit ou une vanne de dosage de celui-ci localisé dans le boîtier.
